# EUROPEAN PATENT APPLICATION

(11) **EP 1 306 094 A1**
(43) Date of publication of application: **02.05.2003**
(21) Application number: 01984401.8
(22) Date of filing: 30.07.2001
(51) Int. Cl.: A61K 45/00, A61K 31/235, A61K 31/704, A61P 43/00, A61P 3/04, A61P 1/16, C07C 69/76

(54) **PREVENTIVES OR REMEDIES FOR OBESITY OR FATTY LIVER**

(30) Priority: 01.08.2000 JP 2000233072
(71) Applicant: SHIONOGI & CO., LTD., Osaka-shi, Osaka 541-0045 (JP)
(72) Inventor: SHIOMI, Teruo, c/o Shionogi & Co., Ltd., Koka-gun, Shiga 520-3423 (JP); HARA, Seijiro, c/o Shionogi & Co., Ltd., Toyonaka-shi, Osaka 561-0825 (JP)
(74) Representative: Baverstock, Michael George Douglas
(86) International application number: JP0106532
(87) International publication number: WO02009757

(57) **Abstract**

The present invention provides a new use of an agent that inhibits bile acid reabsorption. An agent that inhibits bile acid reabsorption is useful for the prevention or treatment of obesity or fatty liver.

## Description

### FIELD OF THE INVENTION

The invention relates to a pharmaceutical composition for prevention or treatment of obesity or fatty liver, and specifically relates to a pharmaceutical composition for prevention or treatment of obesity or fatty liver comprising an inhibitor of bile acid reabsorption, to a method for prevention or treatment of obesity or fatty liver comprising using the inhibitor, and to such a new use of the inhibitor.

### BACKGROUND ART

Since the U.S. LRC-CPPT (Lipid Research Clinics Coronary Primary Prevention Trial) reported in 1984 that the medication of hypercholesterolemia utilizing agents that accelerate bile acid excretion reduced the incidence of ischemic heart diseases, diverse drugs that function through such mechanism have been developed to treat hypercholesterolemia. The drugs include inhibitors of bile acid transport proteins, which have been described, for example, as lignan analogues (JP Publication (kokai) No. 310634/1993, USP No. 5420333) and glucuronic acid derivatives thereof (JP Publication (kokai) No. 241206/1997). It has been known that such inhibitors and other compounds that have an activity of inhibiting bile acid reabsorption inhibit the reabsorption of bile acid from the small intestine and thus decrease the LDL-cholesterol level in blood. However, it has never been known that those inhibitors exhibit an anti-obesity effect nor any effect on fatty liver.

### SUMMARY OF THE INVENTION

The present inventors further studied bile acid reabsorption inhibitors to search for agents that decrease the cholesterol level, and found that such inhibitors exhibit the preventive and therapeutic actions on obesity and fatty liver. The present invention is based on the fact that the inventors found the preventive and therapeutic actions of bile acid reabsorption inhibitors on obesity and fatty liver for the first time. Thus, the present invention aims at providing a pharmaceutical composition for preventing or treating obesity or fatty liver which comprises an inhibitor of bile acid reabsorption; a method for preventing or treating obesity or fatty liver in mammals suffering from obesity or fatty liver, which comprises administering an inhibitor of bile acid reabsorption in an amount effective for preventing or treating obesity or fatty liver to said mammals; and a use of an inhibitor of bile acid reabsorption for manufacturing a medicament for preventing or treating obesity or fatty liver.

### BRIEF DESCRIPTION OF THE DRAWING

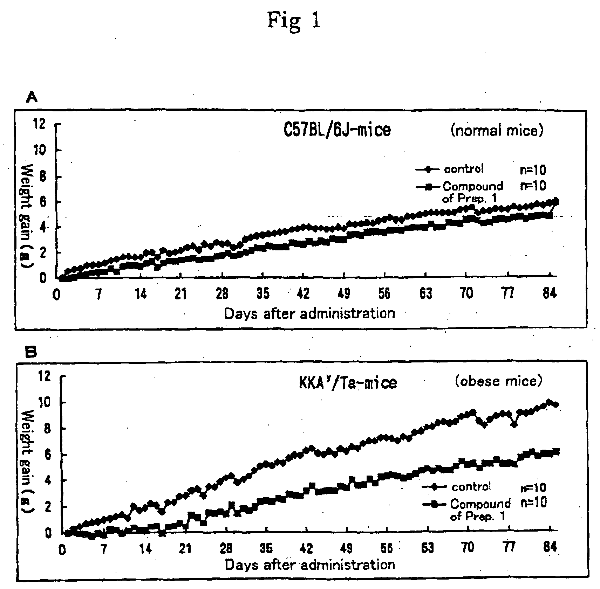

Figure 1 is a graph showing the increase in body weight of mice receiving the bile acid reabsorption inhibitor compared with that of the control. "A" shows the results of normal mice, whereas "B" shows the results of obese mice.

### DETAILED DESCRIPTION OF THE INVENTION

As the first aspect, the present invention provides a pharmaceutical composition for preventing or treating obesity or fatty liver which comprises an inhibitor of bile acid reabsorption.

As used herein, the term "obesity" means a pathology wherein excessive amounts of fat accumulate in the body. In general, obese persons are more likely to have various diseases, and are at higher risk for glucose metabolism dysfunction, cardiovascular disorders, hypertension or the like than non-obese persons. As used herein, the term "fatty liver" means a pathology wherein triglyceride levels in liver are elevated due to various causal factors compared to those in the normal liver. Since the existence of non-alcoholic fatty hepatitis and hepatitis C induce efficiently lipogenesis, fatty liver has attracted interest.

As used herein, inhibitors of bile acid reabsorption mean any agent that inhibits bile acid reabsorption so as to suppress the enterohepatic circulation enabling the recycling of bile acid.

Inhibitors of bile acid reabsorption, as used herein, include lignan analogues and glucuronic acid derivatives thereof, benzothiazepine derivatives, basic anionic ion exchange resins, nonabsorbable aqueous gels, and cationic natural high-molecular compounds.

Specific inhibitors of bile acid reabsorption of lignan analogue and glucuronic acid derivatives thereof are described in JP Patent No. 2839805 and JP Publication (kokai) No. 241206/1997, and are exemplified by a compound of formula (I): 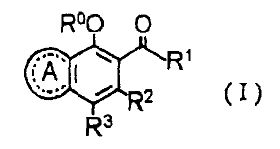 wherein
R⁰ is hydrogen or a hydrophilic group;
R¹ is a lower alkyl group which may be optionally substituted, a cycloalkyl group which may be optionally substituted, a cycloalkyl-lower alkyl group which may be optionally substituted, an aryl group which may be optionally substituted, an aralkyl group which may be optionally substituted, or a heterocyclic group which may be optionally substituted;
R² is a group of the formula: -COOR' wherein R' is a lower alkyl group which may be optionally substituted or an aralkyl group which may be optionally substituted, a lower alkyl group, or a halogenated lower alkyl, or R¹ and R² are combined together to form trimethylene;
R³ is a phenyl group which may be optionally substituted; and
ring A is a benzene ring which may be optionally substituted, or a heterocyclic ring which contains S or O, and which may be optionally substituted; and a pharmaceutically acceptable salt or hydrate thereof.

The term "lower alkyl which may be optionally substituted" means a straight or branched chain C1-C6 alkyl group that may have one or more substituent(s), and includes methyl, ethyl, n-propyl, i-propyl, n-butyl, i-butyl, s-butyl, t-butyl, n-pentyl, i-pentyl, neopentyl, s-pentyl, 1-ethylpropyl, n-hexyl, neohexyl, i-hexyl, and s-hexyl. The term "cycloalkyl group which may be optionally substituted" means a C3-C7 cycloalkyl that may have one or more substituent(s). Examples of C3-C7 cycloalkyl include cyclopropane, cyclobutane, cyclopentane, cyclohexane, and cycloheptane. The term "cycloalkyl-lower alkyl group which may be optionally substituted" means a lower alkyl group as defined above substituted with a cycloalkyl which may have a substituent as defined above, and includes cyclopropylmethyl, cyclobutylpropyl, cyclopentylethyl, cyclohexylpropyl, and cyclohexylmethyl. Substituent on the groups defined above may be optionally selected from a group consisting of a lower alkyl group as defined above, a hydroxyl group, a halogen atom (F, Cl, Br, I), an amino group, or a lower alkoxy group. Examples of a lower alkoxy group include methoxy, ethoxy, n-propoxy, i-propoxy, n-buthoxy, i-buthoxy, s-buthoxy, t-buthoxy, n-pentyloxy, i-pentyloxy, neopentyloxy, s-pentyloxy, n-hexyloxy, neohexyloxy, i-hexyloxy, and s-hexyloxy.

The term "aryl group which may be optionally substituted" means a phenyl or naphthyl which may have one or more substituent(s). Such substituents may be optionally selected from a group consisting of a lower alkyl group as defined above, a lower alkoxy group as defined above, a hydroxyl group, a halogen atom (F, Cl, Br, I), a halogenated alkyl group such as trifluoromethyl, and an amino group The term "aralkyl group which may be optionally substituted" means a lower alkyl group as defined above substituted with an aryl group which may be optionally substituted as defined above, and includes benzyl, p-methoxybenzyl, phenethyl, phenylpropyl, and naphthylmethyl The term "heterocyclic group which may be optionally substituted" means a 5-7-membered aromatic or non-aromatic heterocyclic group containing at least one heteroatom of N, S or O, which may have one or more substituent(s). Examples of an aromatic heterocyclic group include pyrrole, imidazole, pyrazole, pyridine, pyridazine, pyrimidine, pyrazine, isoxazole, oxazole, 1,2,3-triazine, 1,2,4-triazine, thiazole, isothiazole, 1,2,3-thiadiazole, furan, and thiophene. Examples of a non-aromatic heterocyclic group include piperidine, morpholine, pyrroline, tetrahydrothiazine, and tetrahydropyran. Preferred heterocyclic groups include a saturated heterocyclic group containing one N atom, and are exemplified by piperidine or the like. Examples of such substituents include a lower alkyl which may be optionally halogenated, a lower alkoxy which may be optionally halogenated, a lower alkoxycarbonyl which may be optionally halogenated, a lower alkanoyl which may be optionally halogenated, a lower alkyl sulfonyl which may be optionally halogenated, a lower alkoxy sulfonyl which may be optionally halogenated, carboxyl, hydroxy, halogen, amino, and amide.

The term "halogenated lower alkyl group" as defined in R² means a lower alkyl group as defined above substituted with a halogen, such as trifluoromethyl, pentafluoroethyl, and chloroethyl.

The term "phenyl group which may be optionally substituted" as defined in R³ means a phenyl group which may have one or more substituent(s), or a phenyl group-containing condensed ring such as 3,4-ethylenedioxyphenyl group. Such substituents may be optionally selected from a group consisting of a lower alkyl group as defined above, a lower alkoxy group as defined above, a hydroxyl group, and a halogen atom, with a lower alkoxy group being preferred.

The term "benzene ring which may be optionally substituted" as defined in ring A means a benzene ring which may have one or more substituent(s). Such substituents may be optionally selected from a group consisting of a lower alkyl group as defined above, a lower alkoxy group as defined above, a hydroxyl group, a halogen atom, and an alkylenedioxy group (methylenedioxy, ethylenedioxy, and the like), with a lower alkoxy group being preferred. The term "a heterocyclic ring which contains S or O, and which may be optionally substituted" means a 5-6-membered aromatic heterocyclic group containing O or S within the ring, which may have one or more substituent(s). Examples of the aromatic heterocyclic group include furan, and thiophene. Such substituents may be optionally selected from a group consisting of a lower alkyl group as defined above, a lower alkoxy group as defined above, a hydroxyl group, a halogen atom (F, Cl, Br, I), and a C1-C3 alkylenedioxy group.

The term "a hydrophilic group" as defined in R⁰ means a combined group having a polar functional group capable of strongly interacting with water, and include an alkyl group (preferably a C1-C10 alkyl group, more preferably a C1-C6 alkyl group), and a cycloalkyl group (preferably a C3-C7 cycloalkyl group), both of which are substituted with one or more substituent(s) that are the same or different, and that are selected from a group consisting of -OH, -COOH -NH₂, -CN, -NHCONH₂, -NHCOCH₃, -SO₃H, -OSO₃H, -CONH₂, -(OCH₂CH₂)n-, and a phosphate group. Such alkyl group and cycloalkyl group are may be intervened with an O atom, an N atom, a S atom, -CONH or the like. Specific examples include a glucuronic acid residue, carboxymethyl, carboxymethylcarbamoylmethyl, dihydroxypropyl, 3-carboxy-3-hydroxypropyl, and glucopyranosyl.

Among the compounds of formula (I), preferred compounds are of formula (II): 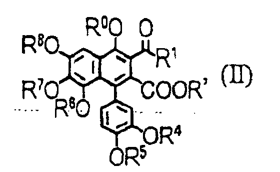 wherein R⁰, R¹ and R' are as defined above, and R⁴, R⁵, R⁶, R⁷ and R⁸ are a lower alkyl, and more preferred compounds are those of the following: 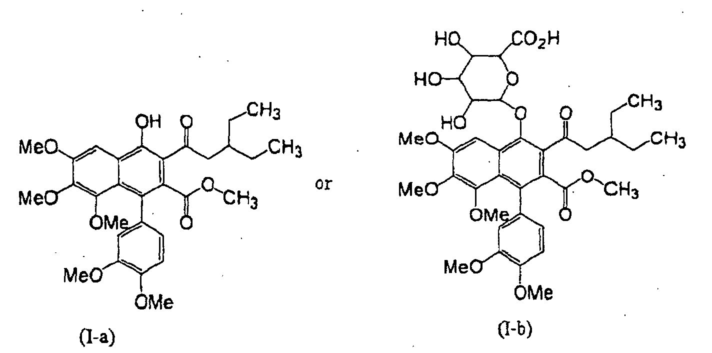 I-a: Methyl 1-(3,4-dimethoxyphenyl)-3-(3-ethylvalelyl)-4-hydroxy-6,7,8-trimethoxy-2-naphthoate; and
I-b: [1-0-[4-(3,4-Dimethoxyphenyl)-2-(3-ethylpentanoyl)-5,6,7-trimethoxy-3-(methoxycarbonyl)naphthalen-1-yl]-β-D-glucopyranoside]uronic acid

The compounds of formulas (I) and (II) may be prepared in accordance with the processes described in JP Patent No. 2839805 and JP Publication (kokai) No. 241206/1997, which are incorporated herein by this reference.

Pharmaceutically acceptable salts of the compounds of formula (I) include alkali metal salts such as a sodium salt and a potassium salt, alkali earth metal salts such as a calcium salt and a magnesium salt, quaternary ammonium salts such as a tetramethyl ammonium salt, salts formed with an organic base such as a diethylamine salt, inorganic acid addition salts such as a hydrochloride and a sulfate, and organic acid addition salts such as acetate, oxalate and benzenesulfonate. The compounds (I) may be administered orally or parenterally, and the oral administration is preferred. The compounds may be orally administered in a solid form such as tablets, powders, capsules, and granules, which may comprise any additives commonly employed in the art, such as excipients, binders, diluents, and lubricants. Also, the compounds may be administered in liquid forms such as aqueous or oily suspensions, solutions, syrups, and elixirs. Alternatively, the compounds may be parenterally administered in a form of injectable solution. Doses vary depending on, for example, the age and weight, the disease and condition to be treated in a patient, and a method for administration, and cannot be uniformly defined. A typical daily dose for adults, however, may range about 0.1 to about 50 mg per kg.

Further, the following compound may exemplify benzothiazepine derivatives as inhibitors of bile acid reabsorption: 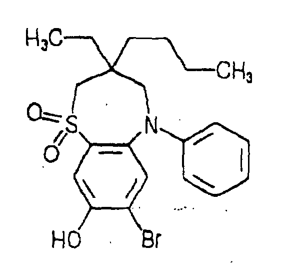 7-Bromo-3-butyl-3-ethyl-8-hydroxy-5-phenyl-1,5-benzothiazepine-1,1-dioxide (213th ACS, San Francisco: MEDI 103, Apr. 1997). It is usual that the compound is orally administered. Doses vary depending on, for example, the age and weight, the disease and condition to be treated in a patient, and a method for administration, and cannot be uniformly defined. A typical daily dose for adults, however, may range about 0.1 to about 50 mg per kg.

The following compounds may exemplify basic anionic ion exchange resins as inhibitors of bile acid reabsorption: 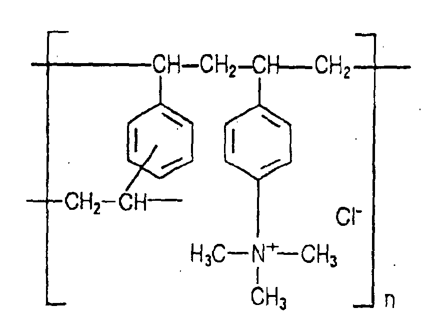 Polystyrene benzyl trimethyl ammonium chloride (cholestyramine) (Kiso to Rinsho 16: 1969, 1982). It is usual that the compound is orally administered. Doses vary depending on, for example, the age and weight, the disease and condition to be treated in a patient, and a method for administration, and cannot be uniformly defined. A typical daily dose for adults, however, may range about 10 to about 1000 mg per kg; 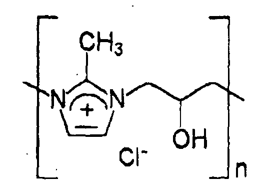 A polymer of 2-methyl-1H-imidazole with (chloromethyl) oxirane (colestilan) (Summary of the 65th The Japanese Pharmacological Society Meeting; No. O-82, March 1992, Sendai). It is usual that the compound is orally administered. Doses vary depending on, for example, the age and weight, the disease and condition to be treated in a patient, and a method for administration, and cannot be uniformly defined. A typical daily dose for adults, however, may range about 10 to about 300 mg per kg; 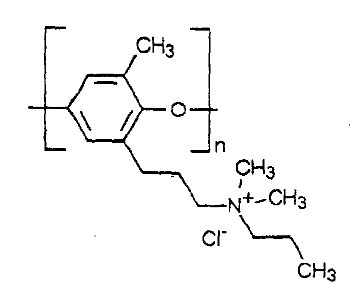 Poly[N,N-dimethyl-N-[1,4-phenylenether-6-methyl-2-propyl]-N-propyl ammonium chloride (KBS-275); and HBS-107 (HISAMITSU PHARMACEUTICAL CO., INC.). It is usual that their compounds are orally administered. Doses vary depending on, for example, the age and weight, the disease and condition to be treated in a patient, and a method for administration, and cannot be uniformly defined. A typical daily dose for adults, however, may range about 10 to about 300 mg per kg.

The following compound may exemplify nonabsorbable aqueous gels as inhibitors of bile acid reabsorption: 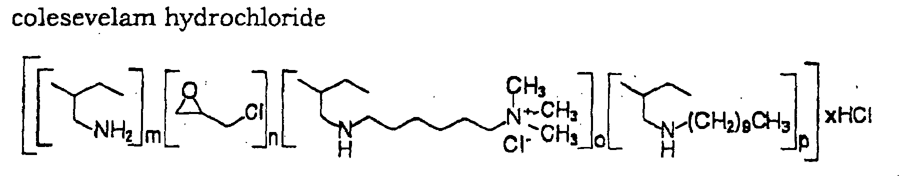 An allyl amine polymer of 1-chloro-2,3-epoxypropane [6-(allyamino)-hexyl] trimethyl ammonium chloride and N-allyldecylamine (colesevelam hydrochloride) (Pharma Market letter 1999/08/09). It is usual that the compound is orally administered. Doses vary depending on, for example, the age and weight, the disease and condition to be treated in a patient, and a method for administration, and cannot be uniformly defined. A typical daily dose for adults, however, may range about 10 to about 300 mg per kg.

Chitosan may exemplify cationic natural high-molecular compounds as inhibitors of bile acid reabsorption. The compound may be administered orally or parenterally in a pharmaceutical form, and the oral administration is preferred. Doses vary depending on, for example, the age and weight, the disease and condition to be treated in a patient, and a method for administration, and cannot be uniformly defined. A typical daily dose for adults, however, may range about 10 to about 300 mg per kg.

The present invention, as the second aspect, provides a method for preventing or treating obesity or fatty liver in mammals suffering from obesity or fatty liver, which comprises administering an inhibitor of bile acid reabsorption as shown above in an amount effective for preventing or treating obesity or fatty liver to said mammals, and as the third aspect, provides a use of an inhibitor of bile acid reabsorption as shown above for manufacturing a medicament for preventing or treating obesity or fatty liver. Specific inhibitors of bile acid reabsorption used in these aspects are as shown above.

Without being limited to a particular theory of mechanism, it is believed that inhibitors of bile acid reabsorption could prevent or treat obesity or fatty liver through decreasing the pool size of bile acid caused by the inhibition of reabsorption of bile acid, which accelerates the lipid absorption, and thus suppressing the lipid absorption.

### BEST MODE FOR CARRYING OUT THE INVENTION

The present invention is further described by the following Preparations and Test Examples, which are not intended to limit the scope of the present invention.

### Preparation 1

### Preparation of methyl 1-(3,4-dimethoxyphenyl)-3-(3-ethylvalelyl)-4-hydroxy-6,7,8-trimethoxy-2-naphthoate

Thirteen mg of p-toluenesulfonic acid was added to a solution of 2.23 g (11.4 mmol) of methyl 6-ethyl-4-oxo-2-octylate that had been prepared as described in JP Patent No. 2839805 and 4.63 g (11.4 mmol) of 2-(3,4-dimethoxy-α-hydroxybenzyl)-3,4,5-trimethoxy benzaldehyde ethylenedioxy acetal in 100 ml of benzene, and the mixture was heated to reflux for one hour. The reaction was concentrated *in vacuo*, and the residue was purified by medium pressure chromatography on silica gel (silica gel 500 g, ethyl acetate : methylene chloride = 1:20) and further by crystallization from diisopropyl ether to yield 1.84 g (29.8%) of the title compound.

### Preparation 2

### Preparation of [1-0-[4-(3,4-dimethoxyphenyl)-2-(3-ethylpentanoyl)-5,6,7-trimethoxy-3-(methoxycarbonyl)naphthalen-1-yl]-β-D-glucopyranoside]uronic acid

1) 95% Silver carbonate (2.12 g, 7.30 mmol) was added to a solution of the compound as prepared in Preparation 1 (7.57 g, 14.0 mmol) in 56 ml of quinoline, and the mixture was stirred for 50 minutes at room temperature, followed by adding thereto 3.97 g (10.0 mmol) of the following compound as described in J. Am. Chem. Soc., 77, 3310 (1955): 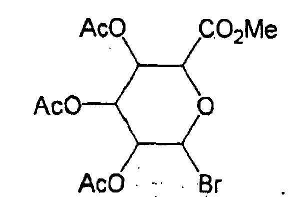 After stirred at the same temperature further for three hours, the reaction was poured into an ice-water, and extracted with ethyl acetate. The extract was washed with water, dried over anhydrous magnesium sulfate, and evaporated *in vacuo*. The residue was purified by chromatography on silica gel (n-C₆H₁₄ :EtOAc = 1:1) to yield 6.24 g (72.8%) of the glucuronic acid conjugate methyl ester triacetate of the compound of Preparation 1.
   ¹H-NMR: δ (CDCl₃) 0.80-0.96 (6H, m), 1.28-1.56 (4H, m), 1.81-1.95 (1H, m), 2.01 (3H, s), 2.05 (3H, s), 2.73-2.92 (1H, m), 3.01-3.15 (1H, m), 3.26 and 3.28 (3H, each s), 3.41 and 3.42 (3H, each s), 3.72 and 3.82 (3H, each s), 3.82 and 3.86 (3H, each s), 3.88 (3H, s), 3.91 and 3.93 (3H, each s), 4.05 (3H, s), 5.10 (1H, dd, J=1.0Hz, 7.8Hz), 5.20-5.35 (2H, m), 5.39-5.51 (1H,m), 6.73-6.93 (3H, m), 7.38 and 7.42 (1H, each s).
2) 60% Sodium hydride (57 mg, 1.4 mmol) was added to a solution of the compound as prepared in the previous reaction (6.00 g, 7.00 mmol) in 60 ml of methanol, and the mixture was stirred for three hours at room temperature. The reaction was poured into a diluted hydrochloric acid under ice-cooling, and extracted with ethyl acetate. The extract was washed with water, dried over anhydrous magnesium sulfate, and evaporated *in vacuo*. The residue was purified by chromatography on silica gel (EtOAc) to yield 3.94 g (77%) of the methyl ester of interest.
3) A 0.1 N aqueous solution of sodium hydroxide (9.03 ml) was added to a solution of the methyl ester as prepared in the previous reaction (660 mg, 0.903 mmol) in 50 ml of methanol under ice-cooling, and the mixture was stirred for two hours at room temperature. To the residue obtained by the evaporation of the reaction, 9.03 ml of 0.1 N hydrochloric acid was added, and the mixture was extracted with ethyl acetate. The extract was washed with water, dried over anhydrous magnesium sulfate, and evaporated *in vacuo*. The residue was purified by chromatography on silica gel (AcOH : EtOAc = 1:30), and further by chromatography on silica gel (CH₂ Cl₂ : CH₃ CN : AcOH = 12:8:1) to yield 242 mg (37%) of the glucuronic acid conjugate of the compound of Preparation 1.
   ¹H-NMR: δ (CDCl₃) 0.81 and 0.88 (6H, each t, J=7.4 and 7.1Hz), 1.15-1.43 (4H, m), 1.75-1.91 (1H, m), 2.70 and 2.79 (1H, each d, J=6.6 and 6.8Hz), 3.17 (3H, s), 3.20-3.56 (4H, m), 3.26 and 3.28 (3H each s), 3.66 and 3.71 (3H, each s), 3.75 and 3.82 (6H, m), 3.96 (3H, s) 4.69 and 4.73 (1H, each d, J=5.8 and 6.0Hz), 5.35 (1H, d, J=5.0Hz, OH) 6.16-6.25 (1H, m, OH), 6.54-6.97 (3H, m), 8.17 (1H, s).
   IR: υ (CHCl₃) 3600-3150, 1740, 1694cm⁻¹.

### Test Example 1

### Method

In the test example, each 10 of the male KKA^{y} /Ta Jcl mice and C57BL/6J mice aged 10 weeks were used as obesity model mice and non-obesity normal model mice, respectively. The compound of Preparation 1 was orally administered to the animals at 10 mg/kg/day for 12 weeks continuously, and the body weights of the animals were daily measured. Four hours after the final administration of the compound, the animals were sacrificed, and the levels of triglyceride in the liver tissues were determined.

The compound of Preparation 1 had been suspended in an aqueous solution of 5% gum arabic (Nakarai Chemcial, Lot. M5E5329) to provide a 0.1% suspension for use. As a control, only a vehicle (5% aqueous gum arabic solution) was administered as well, and the triglyceride level was also determined.

The results were shown in Figure 1. The non-obesity normal mice receiving the compound showed the lower rate of increase in body weight than that of the control, but no significant difference was observed between them (Figure 1-A). On the other hand, the compound showed the significant inhibition of weight gain in obesity mice (Figure 1-B).

Further, the animals receiving the compound of Preparation 1 showed the significant decrease in the triglyceride level in the liver tissue compared to the control, as shown in Table 1.

**Table 1**

| (Oral administration for 12 weeks) | | |
|---|---|---|
| | triglyceride (mg/g) | |
| | C57BL/6J mice | KKA^{y} mice |
| Control | 12.0 ± 1.0 | 232.9 ± 12.7 |
| Compound of Prep. 1 | 13.2 ± 1.2 | 74.7 ± 7.4** |

| | | |
|---|---|---|
| **: p < 0.01 (to the control) | | |

### Test Example 2

In a similar way to Test Example 1, the male KKA^{y} /Ta Jcl mice aged 10 weeks were orally administered with the compound of Preparation 1 at 10 mg/kg/day, and fed a diet containing 3% cholestyramine (corresponding to about 4 g/kg/day) for two weeks continuously, and the level of triglyceride in the liver tissue was determined (eight animals per group). Also, the control was administered with only a vehicle (5% aqueous gum arabic solution).

The animals receiving the compound of Preparation 1 and the animals receiving cholestyramine showed the significant decrease in the triglyceride level in the liver tissue compared to the control, as shown in Table 2.

**Table 2**

| (Administration for 2 weeks) | |
|---|---|
| | triglyceride (mg/g) |
| Control | 107.8 ± 10.2 |
| Compound of Prep. 1 | 49.2 ± 7.7** |
| Cholestyramine | 40.7 ± 5.5** |

| | |
|---|---|
| **: p<0.01 (to the control) | |

### INDUSTRIAL APPLICABILITY

Agents that inhibit bile acid reabsorption were found to be useful for the prevention or treatment of obesity or fatty liver. This finding has provided the new therapeutical uses of such agents and other agents known as inhibitors of bile acid reabsorption.

## Claims

1. A pharmaceutical composition for preventing or treating obesity which comprises an inhibitor of bile acid reabsorption.

2. The pharmaceutical composition of claim 1, in which an inhibitor of bile acid reabsorption is a compound of formula (I): 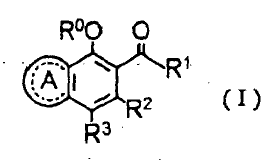 wherein
R⁰ is hydrogen or a hydrophilic group; R¹ is a lower alkyl group which may be optionally substituted, a cycloalkyl group which may be optionally substituted, a cycloalkyl-lower alkyl group which may be optionally substituted, an aryl group which may be optionally substituted, an aralkyl group which may be optionally substituted, or a heterocyclic group which may be optionally substituted; R² is a group of the formula: -COOR' wherein R' is a lower alkyl group which may be optionally substituted or an aralkyl group which may be optionally substituted, a lower alkyl group, or a halogenated lower alkyl, or R¹ and R² are combined together to form trimethylene; R³ is a phenyl group which may be optionally substituted; ring A is a benzene ring which may be optionally substituted, or a heterocyclic ring which contains S or O, and which may be optionally substituted; or a pharmaceutically acceptable salt or hydrate thereof.

3. The pharmaceutical composition of claim 2, in which a compound of formula (I) is a compound of formula (II): 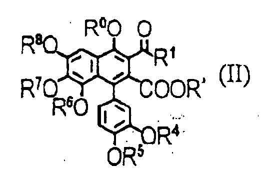 wherein R⁰, R¹ and R' are as defined above, and R⁴, R⁵, R⁶, R⁷ and R⁸ are a lower alkyl.

4. The pharmaceutical composition of claim 1, in which the inhibitor of bile acid reabsorption is methyl 1-(3,4-dimethoxyphenyl)-3-(3-ethylvalelyl)-4-hydroxy-6,7,8-trimethoxy-2-naphthoate, or a pharmaceutically acceptable salt or hydrate thereof.

5. The pharmaceutical composition of claim 1, in which the inhibitor of bile acid reabsorption is [1-0-[4-(3,4-dimethoxyphenyl)-2-(3-ethylpentanoyl)-5,6,7-trimethoxy-3-(methoxycarbonyl)naphthalen-1-yl]-β-D-glucopyranoside]uronic acid, or a pharmaceutically acceptable salt or hydrate thereof.

6. A method for preventing or treating obesity in mammals suffering from obesity, which comprises administrating an inhibitor of bile acid reabsorption in an amount effective for preventing or treating obesity to said mammals.

7. The method for preventing or treating obesity according to claim 6, in which an inhibitor of bile acid rcabsorption is a compound of formula (I) as defined in claim 2.

8. The method for preventing or treating obesity according to claim 6, in which the inhibitor of bile acid reabsorption is methyl 1-(3,4-dimethoxyphenyl)-3-(3-ethylvalelyl)-4-hydroxy-6,7,8-trimethoxy-2-naphthoate, or a pharmaceutically acceptable salt or hydrate thereof.

9. The method for preventing or treating obesity according to claim 6, in which the inhibitor of bile acid reabsorption is [1-0-[4-(3,4-dimethoxyphenyl)-2-(3-ethylpentanoyl)-5,6,7-trimethoxy-3-(methoxycarbonyl)naphthalen-1-yl]-β-D-glucopyranoside]uronic acid, or a pharmaceutically acceptable salt or hydrate thereof.

10. Use of an inhibitor of bile acid reabsorption for manufacturing a medicament for preventing or treating obesity.

11. The use of claim 10, in which an inhibitor of bile acid reabsorption is a compound of formula (I) as defined in claim 2.

12. The use of claim 10, in which the inhibitor of bile acid reabsorption is methyl 1-(3,4-dimethoxyphenyl)-3-(3-ethylvalelyl)-4-hydroxy-6,7,8-trimethoxy-2-naphthoate, or a pharmaceutically acceptable salt or hydrate thereof.

13. The use of claim 10, in which the inhibitor of bile acid reabsorption is [1-0-[4-(3,4-dimethoxyphenyl)-2-(3-ethylpentanoyl)-5,6,7-trimethoxy- 3-(methoxycarbonyl)naphthalen-1-yl]-β-D-glucopyranoside]uronic acid, or a pharmaceutically acceptable salt or hydrate thereof.

14. A pharmaceutical composition for preventing or treating fatty liver which comprises an inhibitor of bile acid reabsorption.

15. The pharmaceutical composition of claim 14, in which an inhibitor of bile acid reabsorption is a compound of formula (I): 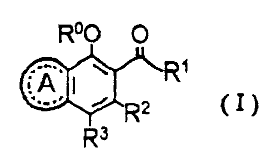 wherein
R⁰ is hydrogen or a hydrophilic group; R¹ is a lower alkyl group which may be optionally substituted, a cycloalkyl group which may be optionally substituted, a cycloalkyl-lower alkyl group which may be optionally substituted, an aryl group which may be optionally substituted, an aralkyl group which may be optionally substituted, or a heterocyclic group which may be optionally substituted; R² is a group of the formula: -COOR' wherein R' is a lower alkyl group which may be optionally substituted or an aralkyl group which may be optionally substituted, a lower alkyl group, or a halogenated lower alkyl, or R¹ and R² are combined together to form trimethylene; R³ is a phenyl group which may be optionally substituted; ring A is a benzene ring which may be optionally substituted, or a heterocyclic ring which contains S or O, and which may be optionally substituted; or a pharmaceutically acceptable salt or hydrate thereof.

16. The pharmaceutical composition of claim 15, in which a compound of formula (I) is a compound of formula (II): 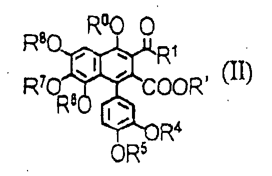 wherein R⁰, R¹ and R' are as defined above, and R⁴, R⁵, R⁶, R⁷ and R⁸ are a lower alkyl.

17. The pharmaceutical composition of claim 14, in which the inhibitor of bile acid reabsorption is methyl 1-(3,4-dimethoxyphenyl)-3-(3-ethylvalelyl)-4-hydroxy-6,7,8-trimethoxy-2-naphthoate, or a pharmaceutically acceptable salt or hydrate thereof.

18. The pharmaceutical composition of claim 14, in which the inhibitor of bile acid reabsorption is [1-0-[4-(3,4-dimethoxyphenyl)-2-(3-ethylpentanoyl)-5,6,7-trimethoxy-3-(methoxycarbonyl)naphthalen-1-yl]-β-D-glucopyranoside]uronic acid, or a pharmaceutically acceptable salt or hydrate thereof.

19. A method for preventing or treating fatty liver in mammals suffering from fatty liver, which comprises administrating an inhibitor of bile acid reabsorption in an amount effective for preventing or treating fatty liver to said mammals.

20. The method for preventing or treating fatty liver according to claim 19, in which an inhibitor of bile acid reabsorption is a compound of formula (I) as defined in claim 15.

21. The method for preventing or treating fatty liver according to claim 19, in which the inhibitor of bile acid reabsorption is methyl 1-(3,4-dimethoxyphenyl)-3-(3-ethylvalelyl)-4-hydroxy-6,7,8-trimethoxy-2-naphthoate, or a pharmaceutically acceptable salt or hydrate thereof.

22. The method for preventing or treating fatty liver according to claim 19, in which the inhibitor of bile acid reabsorption is [1-0-[4-(3,4-dimethoxyphenyl)-2-(3-ethylpentanoyl)-5,6,7-trimethoxy-3-(methoxycarbonyl)naphthalen-1-yl]-β-D-glucopyranoside]uronic acid, or a pharmaceutically acceptable salt or hydrate thereof.

23. Use of an inhibitor of bile acid reabsorption for manufacturing a medicament for preventing or treating fatty liver.

24. The use of claim 23, in which an inhibitor of bile acid reabsorption is a compound of formula (I) as defined in claim 15.

25. The use of claim 23, in which the inhibitor of bile acid reabsorption is methyl 1-(3,4-dimethoxyphenyl)-3-(3-ethylvalelyl)-4-hydroxy-6,7,8-trimethoxy-2-naphthoate, or a pharmaceutically acceptable salt or hydrate thereof.

26. The use of claim 23, in which the inhibitor of bile acid reabsorption is [1-0-[4-(3,4-dimethoxyphenyl)-2-(3-ethylpentanoyl)-5,6,7-trimethoxy-3-(methoxycarbonyl)naphthalen-1-yl]-β-D-glucopyranoside]uronic acid, or a pharmaceutically acceptable salt or hydrate thereof.
